# EUROPEAN PATENT APPLICATION

(11) **EP 4 759 404 A1**
(43) Date of publication of application: **17.06.2026**
(21) Application number: 24851362.4
(22) Date of filing: 27.05.2024
(51) Int. Cl.: B01J 8/04, C07C 1/12, C07C 9/04

(54) **GENERATOR**

(30) Priority: 08.08.2023 JP 2023129551
(71) Applicant: Kanadevia Corporation, Osaka-shi, Osaka 559-8559 (JP); Kanadevia Inova AG, 8005 Zürich (CH)
(72) Inventor: SENN, Patrick, 8005 Zürich (CH)
(74) Representative: Grünecker Patent- und Rechtsanwälte PartG mbB
(86) International application number: PCT/JP2024/019378
(87) International publication number: WO 2025/032928

(57) **Abstract**

A novel generator that generates a product gas is provided. The generator includes a first reactor having a plate-type heat exchange surface therein, the first reactor being configured to generate a product gas by a catalytic reaction of a feed gas and to discharge the feed gas and the product gas, a first circulation pathway through which a first heat medium that passes through the first reactor to adjust temperature inside of the first reactor circulates, a second reactor having a tube-type heat exchange surface therein, the second reactor being configured to generate the product gas by a catalytic reaction of the feed gas discharged from the first reactor and to discharge the product gas, and a second circulation pathway through which a second heat medium that passes through the second reactor to adjust temperature inside of the second reactor circulates.

## Description

### TECHNICAL FIELD

The present invention relates to a generator.

### BACKGROUND ART

A feed gas containing carbon dioxide and hydrogen is supplied to a reactor, and a product gas is generated from the feed gas by an exothermic reaction of the feed gas. For example, PTL 1 discloses a plate-type reactor.

### CITATION LIST

### PATENT LITERATURE

PATENT LITERATURE 1: Japanese Translation of PCT Application No. 2022-508538

### SUMMARY OF INVENTION

### TECHNICAL PROBLEM

A product gas and an unreacted feed gas are discharged from a reactor, and the product gas is separated from the unreacted feed gas by using a separation membrane. The unreacted feed gas is resupplied to the reactor by a compressor. Conventional apparatuses need a separation membrane for separating the product gas from the unreacted feed gas and a compressor for resupplying the separated feed gas to the reactor. In this respect, and the conventional apparatuses have room for improvement. Plate-type reactors are expensive. For example, if a plate-type reactor is disposed at the first stage of a pathway for generating a product gas and a plate-type reactor is disposed at the second stage of the pathway in place of a separation membrane and a compressor, the equipment cost will increase, as compared with the case where a separation membrane and a compressor are installed.

The present invention has been made in view of the above circumstances, and an object of the present invention is to provide a novel generator that generates a product gas.

### SOLUTION TO PROBLEM

According to the present invention for solving the above problem, a generator includes a first reactor having a plate-type heat exchange surface therein, the first reactor being configured to generate a product gas by a catalytic reaction of a feed gas and to discharge the feed gas and the product gas, a first circulation pathway through which a first heat medium that passes through the first reactor to adjust temperature inside of the first reactor circulates, a second reactor having a tube-type heat exchange surface therein, the second reactor being configured to generate the product gas by a catalytic reaction of the feed gas discharged from the first reactor and to discharge the product gas, and a second circulation pathway through which a second heat medium that passes through the second reactor to adjust temperature inside of the second reactor circulates. The novel generator that generates a product gas is provided.

The above generator may include a steam drum provided in the first circulation pathway, and the steam drum may discharge steam generated from water contained in the first heat medium by reaction heat inside of the first reactor. In this way, the steam discharged from the steam drum can be utilized. The steam discharged from the steam drum may be utilized as it is. The steam may be utilized to recover CO₂ in the exhaust gas. For example, in a chemical absorption method for recovering CO₂, an exhaust gas containing CO₂ is cooled in an exhaust gas cooling tower, and then brought into contact with an absorbent in an absorption tower so that CO₂ in the exhaust gas is absorbed by the absorbent. The absorbent containing CO₂ is delivered to a regeneration tower, and the absorbent is heated by the steam. As a result, the absorbent releases CO₂ and is regenerated. The regenerated absorbent is returned to the absorption tower and reused. In addition, a steam generator may be driven by the steam discharged from the steam drum, and the electric power generated by the steam generator may be used to power devices, or the electric power generated by the steam generator may be stored in electric power storage equipment.

The first heat medium may circulate through the first circulation pathway by natural convection, and the second heat medium may circulate through the second circulation pathway by driving of a pump provided in the second circulation pathway. Since the first heat medium circulates through the first circulation pathway by natural convection, a pump for circulating the first heat medium and electric power for driving the pump are not needed.

The above generator may include a supply unit configured to supply the first heat medium to the first circulation pathway, and a heat exchanger configured to perform heat exchange between the first heat medium supplied from the supply unit to the first circulation pathway and the second heat medium circulating through the second circulation pathway. For example, when the temperature of the second heat medium circulating through the second circulation pathway is higher than the temperature of the first heat medium supplied to the first circulation pathway, the temperature of the first heat medium supplied to the first circulation pathway rises due to the heat exchange in the heat exchanger. Thus, the first heat medium whose temperature has risen is supplied to the first circulation pathway.

The first heat medium may contain water and water vapor, and the second heat medium may be oil or water. The first reactor may include a plurality of plates each having a heat exchange surface and a first catalyst packed between the plurality of plates. The feed gas may pass between the plurality of plates, and the first heat medium may pass through inside of the plurality of plates. The second reactor may include a shell, one or more tubes covered by the shell and each having a heat exchange surface, and a second catalyst packed inside of the one or more tubes. The feed gas discharged from the first reactor may pass through inside of the one or more tubes, and the second heat medium may pass through inside of the shell.

### ADVANTAGEOUS EFFECTS OF INVENTION

A novel generator that generates a product gas can be provided.

### BRIEF DESCRIPTION OF THE DRAWINGS

[Fig. 1] Fig. 1 illustrates a configuration of a generator according to an embodiment.
[Fig. 2] Fig. 2 is a schematic view of a first reactor.
[Fig. 3] Fig. 3 illustrates a part of an internal configuration of the first reactor.
[Fig. 4] Fig. 4 illustrates a configuration of a second reactor.
[Fig. 5] Fig. 5 illustrates a first example of a second circulation pathway.
[Fig. 6] Fig. 6 illustrates a second example of the second circulation pathway.

### DESCRIPTION OF EMBODIMENTS

Hereinafter, an embodiment of the present invention will be described. The following embodiment is an exemplary embodiment of the present invention, and the technical scope of the present invention is not limited to the following aspects.

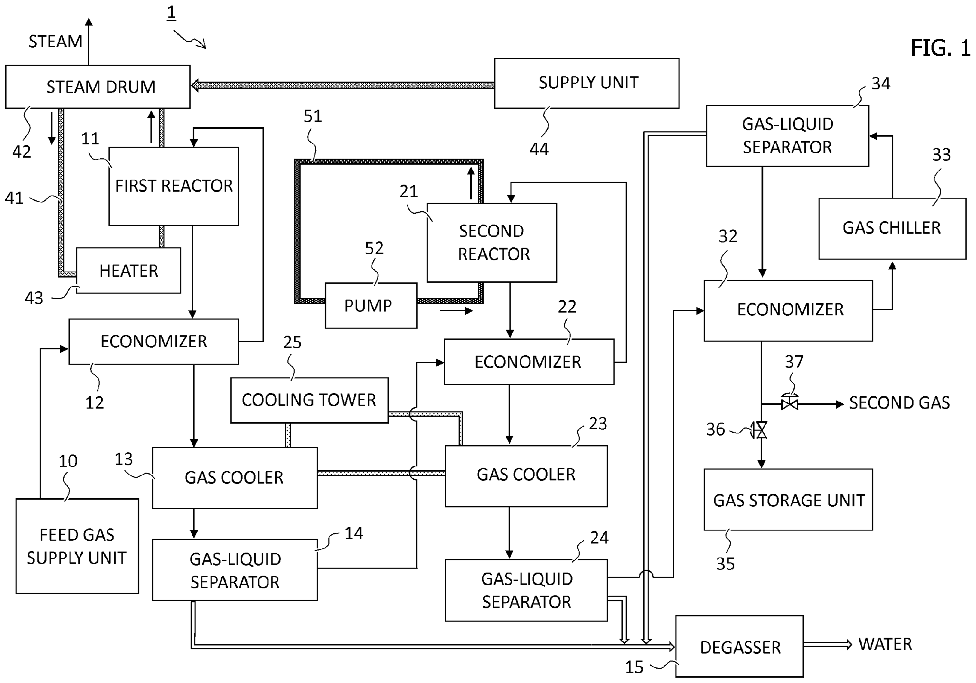

Fig. 1 illustrates a configuration of a generator 1 according to an embodiment. The generator 1 illustrated in Fig. 1 generates, for example, methane gas, as a product gas, and water by an exothermic reaction between gaseous hydrogen and carbon dioxide, which are contained in a feed gas (reactant gas).

The chemical reaction described above is also a reversible reaction. The exothermic reaction described above is expressed by the following chemical reaction formula.

CO₂+ 4H₂→ CH₄+ 2H₂O ... (1)

The generator 1 includes a feed gas supply unit 10, a first reactor 11, an economizer 12, a gas cooler 13, a gas-liquid separator 14, and a degasser 15. The pathways connecting the respective components of the generator 1 include pipes, valves, and the like. The feed gas discharged from the feed gas supply unit 10 passes through the economizer 12 and is supplied to the first reactor 11.

The first reactor 11 is a reactor having a plate-type exchange surface therein, generates a product gas by a catalytic reaction of the feed gas, and discharges the unreacted feed gas and the product gas. The feed gas contains, for example, carbon dioxide (CO₂) and hydrogen (H₂). For example, the stoichiometric ratio of the feed gas supplied from the feed gas supply unit 10 to the first reactor 11 is controlled by flow valves of the feed gas supply unit 10. The flow valves of the feed gas supply unit 10 includes a valve for adjusting the flow rate of carbon dioxide supplied to the first reactor 11 and a valve for adjusting the flow rate of hydrogen supplied to the first reactor 11. The product gas is, for example, methane gas. When the feed gas containing carbon dioxide and hydrogen is supplied to the first reactor 11, the first reactor 11 generates a product gas containing methane by the catalytic reaction between the carbon dioxide and the hydrogen. In addition, the first reactor 11 generates water by the catalytic reaction of the feed gas.

The first reactor 11 discharges a first gas and the generated water (water vapor). The first gas is the product gas, the unreacted feed gas, or a gas mixture of the product gas and the unreacted feed gas. The first gas and the generated water discharged from the first reactor 11 pass through the economizer 12 and flow into the gas cooler 13. In the economizer 12, heat exchange is performed between the feed gas before being supplied to the first reactor 11 and the first gas discharged from the first reactor 11.

The gas cooler 13 is a heat exchanger to cool gas, and condenses the generated water (water vapor) discharged from the first reactor 11. The gas-liquid separator 14 separates the first gas and the generated water (liquid). The generated water separated from the first gas by the gas-liquid separator 14 is delivered to the degasser 15. After gas is removed from the generated water in the degasser 15, the generated water is reused.

The generator 1 includes a second reactor 21, an economizer 22, a gas cooler 23, a gas-liquid separator 24, and a cooling tower 25. The pathways connecting the respective components of the generator 1 include pipes, valves, and the like. The first gas separated from the generated water by the gas-liquid separator 14 passes through the economizer 22 and is supplied to the second reactor 21.

The second reactor 21 is a reactor having a tube-type heat exchange surface therein, generates the product gas by a catalytic reaction of the feed gas, and discharges the product gas. When the feed gas containing carbon dioxide and hydrogen is supplied to the second reactor 21, the second reactor 21 generates a product gas containing methane by the catalytic reaction between the carbon dioxide and the hydrogen. In addition, the second reactor 21 generates water by the catalytic reaction of the feed gas.

The second reactor 21 discharges a second gas and the generated water. The second gas is the product gas or a gas mixture of the product gas and the unreacted feed gas. The first reactor 11 generates a product gas by the catalytic reaction of the feed gas, and discharges the feed gas and the product gas. The second reactor 21 generates a product gas by the catalytic reaction of the feed gas discharged from the first reactor 11, and discharges the product gas. In this way, the generator 1 can generate a highly concentrated product gas. In the generator 1, the first reactor 11 is disposed at the first stage of the pathway (generation pathway) for generating the product gas from the feed gas, and the second reactor 21 is disposed at the second stage of the generation pathway. By disposing the first reactor 11 at the first stage of the generation pathway and disposing the second reactor 21 at the second stage of the generation pathway, the following advantages are obtained, for example.

### (Advantage 1)

By disposing the first reactor 11 at the first stage of the generation pathway and disposing the second reactor 21 at the second stage of the generation pathway, the first gas discharged from the first reactor 11 can be supplied to the second reactor 21. This configuration eliminates the need for installing a separation membrane for separating the product gas and the unreacted feed gas contained in the first gas discharged from the first reactor 11. The equipment cost of separation membranes is high. However, since this configuration eliminates the need for installing a separation membrane, the equipment cost can be reduced. Further, it is not necessary to install a supply compressor for resupplying the unreacted feed gas to the first reactor 11. Thus, the equipment cost and the electric power for driving the supply compressor can be reduced.

### (Advantage 2)

By disposing the first reactor 11 at the first stage of the generation pathway, a high space velocity can be realized. As the space velocity increases, the concentration of the product gas contained in the first gas discharged from the first reactor 11 decreases. The second reactor 21 is disposed at the second stage of the generation pathway, and the first gas discharged from the first reactor 11 is supplied to the second reactor 21. The second reactor 21 generates a product gas from the feed gas contained in the first gas. In this way, the generator 1 can generate a highly concentrated product gas. Therefore, a decrease in the concentration of the product gas contained in the first gas discharged from the first reactor 11 is permissible.

### (Advantage 3)

Plate-type reactors have high heat transfer efficiency and heat recovery capability. However, plate-type reactors are expensive because the plate-type reactors have a complex plate structure in which high-pressure steam circulates. For example, if a plate-type reactor is disposed at the first stage of the generation pathway, and another plate-type reactor is disposed at the second stage of the generation pathway, the equipment cost will increase. On the other hand, shell-and-tube-type reactors are compact and have low equipment costs. By disposing the first reactor 11 at the first stage of the generation pathway and disposing the second reactor 21 at the second stage of the generation pathway, the heat transfer efficiency and heat recovery capability of the generator 1 can be improved. Further, the generator 1 can be downsized, and its equipment cost is reduced.

### (Advantage 4)

The equipment cost is lower when the first reactor 11 is disposed at the first stage of the generation pathway and the second reactor 21 is disposed at the second stage of the generation pathway, compared with the case where the shell-and-tube-type reactor, the separation membrane, and the supply compressor are disposed in the generation pathway. Therefore, by disposing the first reactor 11 at the first stage of the generation pathway and disposing the second reactor 21 at the second stage of the generation pathway, the equipment cost can be reduced.

### (Advantage 5)

The generator 1 is not configured to control the unreacted feed gas, which is contained in the first gas discharged from the first reactor 11, to be resupplied to the first reactor 11. That is, the generator 1 is a once-through system, and a control operation for generating a product gas from the feed gas is easily executed. On the other hand, a system in which the product gas and the unreacted feed gas discharged from the plate-type reactor are separated by a separation membrane is a recycle system. In the recycle system, since the feed gas separated by the separation membrane is resupplied to the plate-type reactor, the gas composition of the feed gas (supplied gas) supplied to the plate-type reactor could change, and this makes it difficult to control the stoichiometric ratio of the feed gas. In the generator 1, since there is no mixing of the feed gas supplied to the first reactor 11 and the unreacted feed gas contained in the first gas, the stoichiometric ratio of the feed gas (CO₂ and H₂) supplied to the first reactor 11 by using the flow valves of the feed gas supply unit 10 can be more easily controlled.

The second gas and the generated water discharged from the second reactor 21 pass through the economizer 22 and flow into the gas cooler 23. In the economizer 22, heat exchange is performed between the first gas before being supplied to the second reactor 21 and the second gas discharged from the second reactor 21.

The gas cooler 23 is a heat exchanger to cool gas, and condenses the generated water (water vapor) discharged from the second reactor 21. The gas-liquid separator 24 separates the second gas and the generated water (liquid). The generated water separated from the second gas by the gas-liquid separator 24 is delivered to the degasser 15. After gas is removed from the generated water in the degasser 15, the generated water is discharged to the outside.

The cooling tower 25 cools cooling water (refrigerant) for condensing the generated water in the gas coolers 13 and 23. The gas coolers 13 and 23 and the cooling tower 25 are connected by pipes through which the cooling water flows. The cooling water cooled by the cooling tower 25 returns to the cooling tower 25 via the gas coolers 13 and 23. A chiller may be used instead of the cooling tower 25.

The generator 1 includes an economizer 32, a gas chiller 33, a gas-liquid separator 34, a gas storage unit 35, and regulating valves 36 and 37. The pathways connecting the respective components of the generator 1 include pipes, valves, and the like. The second gas separated from the generated water by the gas-liquid separator 24 passes through the economizer 32 and flows into the gas chiller 33. The gas chiller 33 cools the second gas. The second gas cooled by the gas chiller 33 passes through the gas-liquid separator 34 and the economizer 32. The gas-liquid separator 34 separates the second gas and the generated water (liquid). The generated water separated from the second gas by the gas-liquid separator 34 is delivered to the degasser 15. In the economizer 32, heat exchange is performed between the second gas before flowing into the gas chiller 33 and the second gas discharged from the gas chiller 33.

The pipes through which the second gas flows are provided with the regulating valves 36 and 37. The second gas flows into the gas storage unit 35 by opening the regulating valve 36 and closing the regulating valve 37. The second gas is discharged to the outside by closing the regulating valve 36 and opening the regulating valve 37. The gas storage unit 35 stores the second gas. The second gas discharged to the outside may be utilized for combustion. Electric power may be generated by combusting the second gas. A power transmission and distribution system (grid) for transmitting the generated electric power to consumers may be installed next to the generator 1. The combustion heat obtained by combusting the second gas may be utilized within the generator 1, or may be utilized in an external device, at a customer's site, or the like.

The generator 1 includes a first circulation pathway 41, a steam drum 42, a heater 43, and a supply unit 44. The pathways connecting the respective components of the generator 1 include pipes, valves, and the like. A first heat medium that passes through the first reactor 11 to adjust the temperature therein circulates through the first circulation pathway 41. The first heat medium contains water and steam (water vapor).

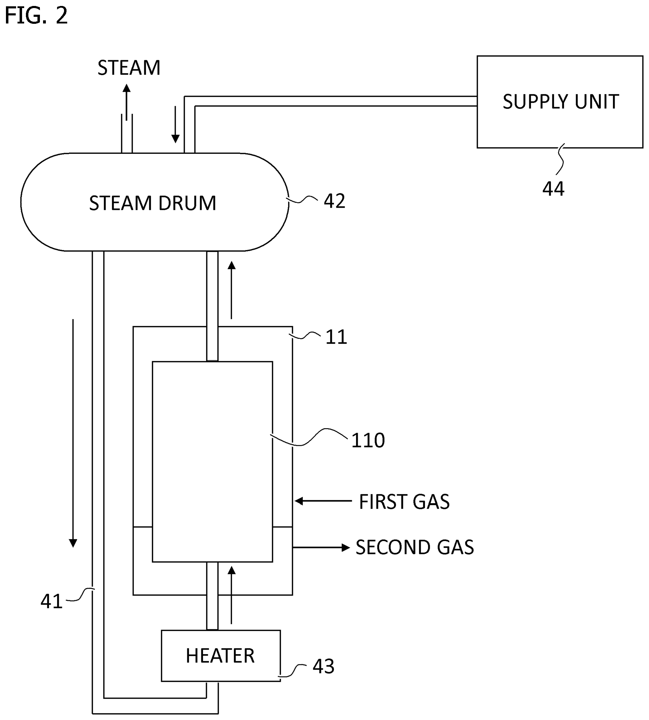

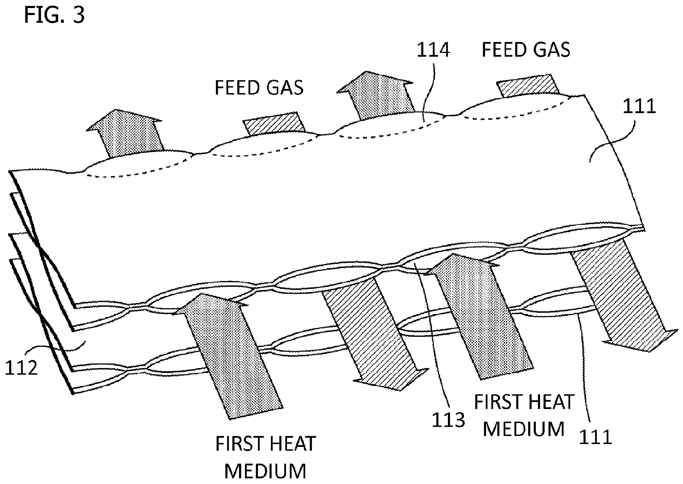

Fig. 2 is a schematic view of the first reactor 11. Fig. 3 illustrates a part of the internal configuration of the first reactor 11. The first reactor 11 is a reactor having a plate-type exchange surface therein. The steam drum 42 is installed above the first reactor 11, and the first heat medium circulating through the first circulation pathway 41 passes through the first reactor 11 and the steam drum 42. The first reactor 11 may be installed such that the direction in which the first heat medium flows is perpendicular (vertical) with respect to the installation surface of the first reactor 11. The first reactor 11 has a plurality of plates 111 each having a heat exchange surface, and a catalyst (first catalyst) packed between the plurality of plates 111. The plurality of plates 111 and the catalyst packed between the plurality of plates 111 are accommodated in a plate package 110 in the first reactor 11.

As illustrated in Fig. 3, the plurality of plates 111 are arranged such that the heat exchange surfaces of the plurality of plates 111 face each other, and the feed gas passes between the plurality of plates 111. The first reactor 11 has a catalyst-packed bed 112 between the plurality of plates 111, and the catalyst-packed bed 112 between the plurality of plates 111 is packed with a catalyst. When the feed gas passes through the catalyst-packed bed 112 between the plurality of plates 111, a product gas is generated by a catalytic reaction of the feed gas.

The first heat medium passes through the inside of the plurality of plates 111. Each of the plates 111 includes an inlet 113 through which the first heat medium flows into each plate 111 and an outlet 114 through which the first heat medium flows out of each plate 111. Each of the plates 111 has a plurality of inlets 113 and a plurality of outlets 114, and a common space is formed inside each of the plates 111. The configuration is not limited to the example illustrated in Fig. 3, and each plate 111 may have one inlet 113 and one outlet 114. The first heat medium flows into each plate 111 through the inlets 113, and flows out of each plate 111 through the outlets 114. Inside of the first reactor 11, reaction heat is generated by the catalytic reaction of the feed gas. When the first heat medium passes through the inside of each plate 111, heat exchange is performed between the first heat medium and a portion (heat generating portion) in which the temperature has risen due to the reaction heat inside of the first reactor 11. In this way, the temperature inside of the first reactor 11 is adjusted. Although it is preferable that the first heat medium and the feed gas be in counter-current flow, the first heat medium and the feed gas may be in cocurrent flow.

In addition, steam is generated from the water contained in the first heat medium by the reaction heat inside of the first reactor 11. A part of the generated steam is delivered to the steam drum 42 connected to the first reactor 11. The steam discharged from the steam drum 42 may be utilized in the generator 1 or may be utilized in an external device or the like. In this way, the steam discharged from the steam drum 42 may be directly (thermally) utilized in another process. The steam discharged from the steam drum 42 may be utilized as it is. The steam may be utilized to recover CO₂ in the exhaust gas. For example, in a chemical absorption method for recovering CO₂, the exhaust gas containing CO₂ is cooled in an exhaust gas cooling tower, and then brought into contact with an absorbent in an absorption tower so that CO₂ in the exhaust gas is absorbed by the absorbent. The absorbent containing CO₂ is delivered to a regeneration tower, and the absorbent is heated by the steam. As a result, the absorbent releases CO₂ and is regenerated. The regenerated absorbent is returned to the absorption tower and reused. A steam generator driven by the steam discharged from the steam drum 42 may be provided inside of the generator 1 or may be provided outside of the generator 1. The steam generator generates electric power by the steam supplied from the steam drum 42. The electric power generated by the steam generator can be used to power devices such as a control unit for operating a process, heating equipment, and a compressor. Further, not only using the electric power generated by the steam generator to power such devices, but the surplus electric power may also be supplied to any power drive. Further, electric power storage equipment may be provided, and the electric power generated by the steam generator may be stored in this electric power storage equipment. According to the generator 1, by utilizing the steam discharged from the steam drum 42, the heat recovery from the first heat medium circulating through the first circulation pathway 41 can be easily performed. By directly (thermally) utilizing the steam discharged from the steam drum 42 in another process, the heat recovery from the first heat medium can be performed more efficiently than by utilizing the steam by using the steam generator.

The heater 43 is a heater that heats the first heat medium circulating through the first circulation pathway 41. The first heat medium heated by the heater 43 passes through the first reactor 11. The heater 43 may heat the first heat medium when the first reactor 11 is started up. After the operation of the first reactor 11 is started, the heater 43 may stop heating the first heat medium. If it there is no need to heat the first heat medium when the first reactor 11 is started up, the installation of the heater 43 may be omitted.

Steam is generated from the water contained in the first heat medium by the reaction heat inside of the first reactor 11. The steam generated inside of the first reactor 11 rises and flows into the steam drum 42 connected to the first reactor 11. A part of the steam in the steam drum 42 is discharged from the steam drum 42, and another part of the steam in the steam drum 42 returns to water in the steam drum 42 and flows into the first reactor 11. In this way, the first heat medium naturally circulates through the first circulation pathway 41 by natural convection generated by the reaction heat inside of the first reactor 11.

The supply unit 44 supplies the first heat medium to the first circulation pathway 41. The supply unit 44 may supply the first heat medium to the first circulation pathway 41 at regular intervals or may constantly supply the first heat medium to the first circulation pathway 41. The supply unit 44 may control the amount of the first heat medium supplied to the first circulation pathway 41 in accordance with the amount of steam discharged from the steam drum 42.

The generator 1 includes a second circulation pathway 51 and a pump 52. A second heat medium that passes through the second reactor 21 to adjust the temperature therein circulates through the second circulation pathway 51. The second heat medium is oil or water. The second heat medium circulates through the second circulation pathway 51 by driving the pump 52 provided in the second circulation pathway 51. The pump 52 is driven by, for example, electric power supplied from a power supply unit provided in the generator 1.

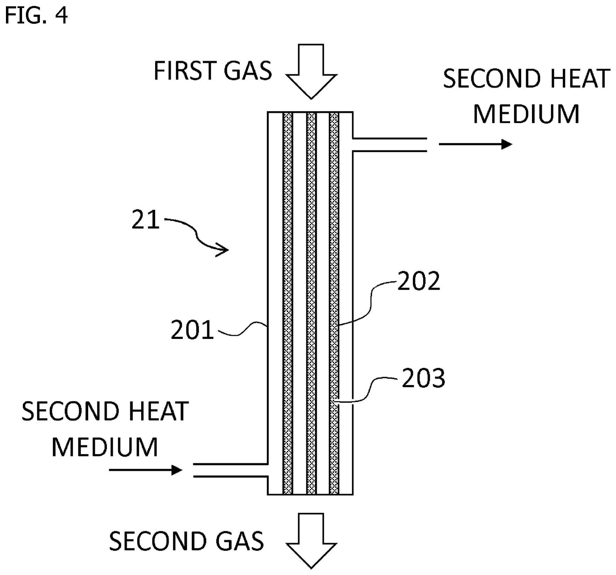

Fig. 4 illustrates a configuration of the second reactor 21. Fig. 4 illustrates a cross section of the second reactor 21. The second reactor 21 includes a shell 201, a plurality of tubes 202 covered by the shell 201 and having a heat exchange surface, and a catalyst (second catalyst) 203 packed inside of the plurality of tubes 202. The individual tube 202 has a cylindrical shape. The shell 201 is provided around the tubes 202. In the configuration example illustrated in Fig. 4, the second reactor 21 has a structure in which the plurality of tubes 202 are provided. However, the configuration is not limited to the example illustrated in Fig. 4, and the second reactor 21 may have a structure in which a single tube 202 is provided. The plurality of tubes 202 extend from one end of the second reactor 21 to the other end of the second reactor 21. In the configuration example illustrated in Fig. 4, the first gas and the second heat medium flow in the vertical direction in the second reactor 21. However, the configuration is not limited to the example illustrated in Fig. 4, and the first gas and the second heat medium may flow in the horizontal direction in the second reactor 21.

The first gas passes through the inside of each tube 202, and the second heat medium passes through the internal space of the shell 201. When the feed gas passes through the inside of each tube 202, the product gas is generated by the catalytic reaction of the feed gas. Inside of the second reactor 21, reaction heat is generated by the catalytic reaction of the feed gas contained in the first gas. When the second heat medium passes through the internal space of the shell 201, heat exchange is performed between the second heat medium and a portion (heat generating portion) in which the temperature has risen due to the reaction heat inside of the second reactor 21. In this way, the temperature inside of the second reactor 21 is adjusted.

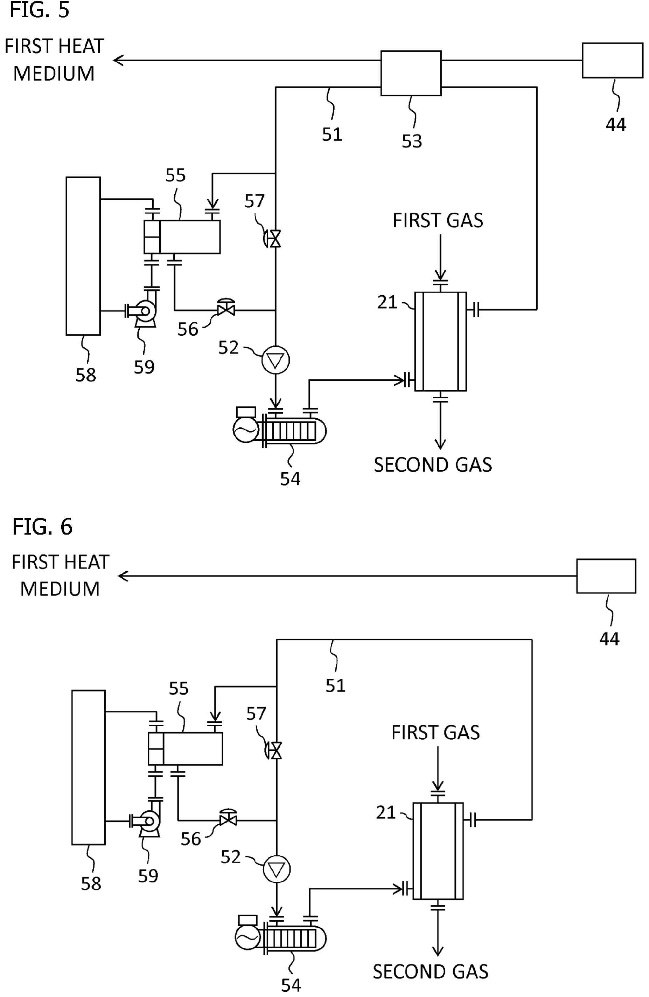

Fig. 5 illustrates a first example of the second circulation pathway 51. In the configuration example illustrated in Fig. 5, the generator 1 includes the second reactor 21, the supply unit 44, the second circulation pathway 51, the pump 52, a heat exchanger 53, and a heater 54. The pathways connecting the respective components of the generator 1 include pipes, valves, and the like. The pump 52, the heat exchanger 53, and the heater 54 are provided in the second circulation pathway 51.

The heat exchanger 53 performs heat exchange between the first heat medium supplied from the supply unit 44 to the first circulation pathway 41 and the second heat medium circulating through the second circulation pathway 51. For example, when the temperature of the second heat medium circulating through the second circulation pathway 51 is higher than the temperature of the first heat medium supplied to the first circulation pathway 41, the temperature of the first heat medium supplied to the first circulation pathway 41 rises due to the heat exchange in the heat exchanger 53. Thus, the first heat medium whose temperature has risen is supplied to the first circulation pathway 41. Normally, the temperature of the second heat medium is higher than the temperature of the first heat medium supplied to the first circulation pathway 41. Thus, the temperature of the first heat medium supplied to the first circulation pathway 41 rises due to the heat exchange in the heat exchanger 53. On the other hand, for example, when the temperature of the first heat medium supplied to the first circulation pathway 41 is higher than the temperature of the second heat medium circulating through the second circulation pathway 51, the temperature of the second heat medium circulating through the second circulation pathway 51 rises due to the heat exchange in the heat exchanger 53.

The heater 54 is a heater that heats the second heat medium circulating through the second circulation pathway 51. The second heat medium heated by the heater 54 passes through the second reactor 21. The heater 54 may heat the second heat medium when the second reactor 21 is started up. After the operation of the second reactor 21 is started, the heater 54 may stop heating the second heat medium.

In the configuration example illustrated in Fig. 5, the generator 1 includes a heat medium cooler 55, regulating valves 56 and 57, a cooling tower 58, and a cooling water circulation pump 59. The pathways connecting the respective components of the generator 1 include pipes, valves, and the like. The heat medium cooler 55 and the regulating valves 56 and 57 are provided in the second circulation pathway 51. The heat medium cooler 55 cools the second heat medium circulating through the second circulation pathway 51 by using cooling water. By opening and closing the regulating valves 56 and 57, the second heat medium having passed through the second reactor 21 can be delivered to the heater 54 via the heat medium cooler 55 or can be delivered to the heater 54 without passing through the heat medium cooler 55. The cooling tower 58 cools the cooling water used in the heat medium cooler 55. The cooling water circulation pump 59 is driven to circulate the cooling water between the heat medium cooler 55 and the cooling tower 58.

The heat of the second heat medium circulating through the second circulation pathway 51 may be utilized for purposes other than the heat exchange with the first heat medium. For example, heat exchange may be performed between the second heat medium and the second gas before flowing into the gas storage unit 35. That is, the second gas before flowing into the gas storage unit 35 may be heated by utilizing the heat of the second heat medium circulating through the second circulation pathway 51. In the configuration example illustrated in Fig. 5, heat exchange is performed between the first heat medium and the second heat medium in the heat exchanger 53 provided in the second circulation pathway 51. However, the configuration is not limited to this example. Heat exchange may be performed between the first heat medium and the second heat medium at any position in the configuration example illustrated in Fig. 5.

Fig. 6 illustrates a second example of the second circulation pathway 51. In the configuration example illustrated in Fig. 6, the heat exchanger 53 is not provided in the second circulation pathway 51. The other components in the configuration example illustrated in Fig. 6 are the same as those in the configuration example illustrated in Fig. 5. In the configuration example illustrated in Fig. 6, heat exchange is not performed between the first heat medium and the second heat medium. By not performing heat exchange between the first heat medium and the second heat medium, the heat of the first heat medium can be utilized only for the heat of the high-temperature and high-pressure steam.

The heat of the second heat medium circulating through the second circulation pathway 51 may be utilized for purposes other than the heat exchange with the first heat medium. For example, heat exchange may be performed between the second heat medium and the second gas before flowing into the gas storage unit 35. That is, the second gas before flowing into the gas storage unit 35 may be heated by using the heat of the second heat medium circulating through the second circulation pathway 51. In the configuration example illustrated in Fig. 6, heat exchange is not performed between the first heat medium and the second heat medium. However, the configuration is not limited to this example. Heat exchange may be performed between the first heat medium and the second heat medium at any position in the configuration example illustrated in Fig. 6.

In addition, each of the processes described above may be understood as a method for generating a product gas in the generator 1 or a method for operating the generator 1. Each of the processes described above may be understood as a generation system or an operation system having at least some of the processes or functions described above. The present invention can be implemented by combining the above means and processes as much as possible.

### REFERENCE SIGNS

1 Generator; 10 Feed gas supply unit; 11 First reactor; 12, 22, 32 Economizer; 13, 23 Gas cooler; 14, 24, 34 Gas-liquid separator; 15 Degasser; 21 Second reactor; 25 Cooling tower; 33 Gas chiller; 35 Gas storage unit; 36, 37 Regulating valve; 41 First circulation pathway; 42 Steam drum; 43 Heater; 44 Supply unit; 51 Second circulation pathway; 52 Pump;

## Claims

1. A generator comprising:
a first reactor having a plate-type heat exchange surface therein, the first reactor being configured to generate a product gas by a catalytic reaction of a feed gas and to discharge the feed gas and the product gas;
a first circulation pathway through which a first heat medium that passes through the first reactor to adjust temperature inside of the first reactor circulates;
a second reactor having a tube-type heat exchange surface therein, the second reactor being configured to generate the product gas by a catalytic reaction of the feed gas discharged from the first reactor and to discharge the product gas; and
a second circulation pathway through which a second heat medium that passes through the second reactor to adjust temperature inside of the second reactor circulates.

2. The generator according to claim 1, comprising a steam drum provided in the first circulation pathway,
wherein the steam drum discharges steam generated from water contained in the first heat medium by reaction heat inside of the first reactor.

3. The generator according to claim 1, wherein
the first heat medium circulates through the first circulation pathway by natural convection, and
the second heat medium circulates through the second circulation pathway by driving of a pump provided in the second circulation pathway.

4. The generator according to claim 1, comprising:
a supply unit configured to supply the first heat medium to the first circulation pathway; and
a heat exchanger configured to perform heat exchange between the first heat medium supplied from the supply unit to the first circulation pathway and the second heat medium circulating through the second circulation pathway.

5. The generator according to claim 1, wherein
the first heat medium contains water and water vapor, and
the second heat medium is oil or water.

6. The generator according to any one of claims 1 to 5, wherein
the first reactor includes a plurality of plates each having a heat exchange surface and a first catalyst packed between the plurality of plates,
the feed gas passes between the plurality of plates,
the first heat medium passes through inside of the plurality of plates,
the second reactor includes a shell, one or more tubes covered by the shell and each having a heat exchange surface, and a second catalyst packed inside of the one or more tubes,
the feed gas discharged from the first reactor passes through inside of the one or more tubes, and
the second heat medium passes through inside of the shell.
